# EUROPÄISCHE PATENTSCHRIFT

(11) **EP 0 431 406 B1**
(45) Veröffentlichungstag und Bekanntmachung des Hinweises auf die Patenterteilung: **08.06.1994**
(21) Anmeldenummer: 90122292.7
(22) Anmeldetag: 22.11.1990
(51) Int. Cl.: G01N 21/90, B07C 5/34

(54) **Vorrichtung zum Beleuchten eines zu prüfenden Bereiches einer Flasche**
Device for lightening a region of a bottle to be inspected
Dispositif d'éclairage d'une région d'une bouteille à examiner

(30) Priorität: 05.12.1989 CH 4351/89
(43) Veröffentlichungstag der Anmeldung: 12.06.1991
(73) Patentinhaber: ELPATRONIC AG, CH-6300 Zug (CH)
(72) Erfinder: Apter, Robert, CH-8600 Dübendorf (CH); Pau, Louis-Français, F-13600 Ceyreste (FR); Agerskov, Carsten, DK-2860 Soborg (DK); Jacobi, Ulrik, DK-2900 Hellerup (DK); Sloth, Hendrik, DK-2720 Vanlose (DK)

(56) Entgegenhaltungen:
- DE-A- 1 798 044
- DE-A- 3 228 464
- US-A- 3 157 332
- US-A- 3 601 616
- US-A- 3 980 890
- US-A- 4 293 219
- PATENT ABSTRACTS OF JAPAN vol. 7, no. 113 (P-197)(1258) 18 Mai 1983,& JP-A-58 34348

## Beschreibung

Die Erfindung betrifft eine Vorrichtung zum Inspizieren eines im wesentlichen hohlzylindrischen Bereiches von entlang einer Bahn bewegten durchsichtigen Hohlkörpern, wobei diese auf einem Bahnabschnitt einen Inspektionsbereich durchlaufen, mit einem auf einer Seite des Bahnabschnittes befindlichen, auf den Inspektionsbereich gerichteten Lichtempfänger und einer auf der gegenüberliegenden Seite des Bahnabschnittes angeordneten, den Inspektionsbereich bestrahlende, Lichtquellen enthaltende Beleuchtungseinrichtung.

Eine bekannte Vorrichtung dieser Art (US-A-4 691 231 oder DE-A-3228464) dient zum Beleuchten der Seitenwände von Flaschen, die sich auf einem sich kontinuierlich bewegenden Förderer befinden und im Durchlicht inspiziert werden, um Defekte in den Wänden der Flaschen festzustellen. Zu diesem Zweck werden bei der bekannten Vorrichtung sechs Videokameras für die Seitenwände benutzt, und zwar je drei Videokameras für einen unteren Seitenwandbereich und drei Videokameras für einen darüber gelegenen Seitenwandbereich. Die Videokameras können entweder bogenförmig um die Flasche oder linear längs des Flaschenförderers angeordnet sein. Die Kameras sind in der Lage, Daten über verschiedene Höhenbereiche jeweils über den gesamten Umfang der Flaschen zu liefern. Damit werden allerdings jeweils diametral gegenüberlegende Flaschenwandbereiche (Vorder- und Rückseite) gleichzeitig durchleuchtet und einander überlagert. Für jedes Paar von übereinander angeordneten Kameras ist auf der zu den Kameras entgegengesetzten Seite der Flasche eine Lichtquelle vorgesehen, für insgesamt sechs Kameras also insgesamt drei Lichtquellen. Eine zu inspizierende Flasche bewegt sich zwischen dem ersten Kamerapaar und dessen zugeordneter Lichtquelle hindurch und wird dabei aufgenommen, dann zwischen dem zweiten Kamerapaar und dessen zugeordneter Lichtquelle und schliesslich zwischen dem dritten Kamerapaar und dessen zugeordneter Lichtquelle. Damit die Flasche für jedes Kamerapaar etwa gleichmässig beleuchtet wird, ist zwischen Kameras und Lichtquellen eine diffuse, lichtdurchlässige Platte angeordnet.

Bei dieser bekannten Vorrichtung ist die Beleuchtung eine reine Durchlicht-Beleuchtung, bei der jeweils Rückseite und Vorderseite (bezogen auf die Kamera) den Lichtstrahlengang gleichermassen beeinflussen. Eine solche Beleuchtung ist nicht geeignet, einen der Abbildungseinrichtung zugekehrten speziellen Bereich der Flasche, wie zum Beispiel den üblicherweise mit Gewinde versehenen Mündungsbereich, mit ausreichendem und gleichmässigem Kontrast zu beleuchten, um Fehler oder geometrische Strukturen im Glas, wie zum Beispiel die Gewindegänge, sichtbar zu machen. Ein solcher Bereich bedarf zur für die Inspektion notwendigen Abbildung einer intensiven, gleichmässigen und kontrastreichen Beleuchtung, damit in diesem Bereich, in welchem die Flaschenoberfläche aufgrund des Gewindes sehr ungleichmässig ist, innere Defekte sowie äussere Defekte, wie Ablagerungen oder Aenderungen der Glasgeometrie, festgestellt werden können. Weiter ist die reine Durchlicht-Beleuchtung zur Inspektion ungeeignet, wenn die Flasche bis in den Mündungsbereich mit einer im wesentlichen lichtundurchlässigen Flüssigkeit gefüllt ist oder das Glas der Flasche selbst wenig lichtdurchlässig ist. Die bekannte Vorrichtung ist daher auch nur zur Inspektion von Flaschen aus Klar- oder durchsichtigem Glas vorgesehen, die im übrigen im Mündungsbereich auch kein Gewinde aufweisen und in diesem Bereich auch nicht mit lichtundurchlässiger Flüssigkeit gefüllt sein dürfen. Weiter werden bei der bekannten Vorrichtung innere Defekte des Glases kaum beleuchtet, weil von dem in Richtung einer durch die Flaschenlängsachse gehenden Normalen auf die Flaschenoberfläche auftreffenden Licht viel durch Reflexion an der Flaschenoberfläche oder an darauf befindlichem Wasser oder durch Brechung verlorengeht. Der Lichteinfall in der Normalen ist bei der bekannten Vorrichtung vorgegeben, weil sich Lichtquelle und zugeordnete Kamera auf entgegengesetzten Seiten der Flasche stets diametral gegenüberliegen.

Aufgabe der Erfindung ist es, eine Vorrichtung der eingangs genannten Art so auszubilden, dass sie in der Lage ist, einen im wesentlichen hohlzylindrischen Bereich eines durchsichtigen Hohlkörpers mit starkem Kontrast abzubilden, um geometrische Strukturen oder Fehler in der inneren Struktur des Hohlkörpers bei der Inspektion besser sichtbar zu machen.

Diese Aufgabe ist bei einer Vorrichtung nach der Erfindung dadurch gelöst, dass die Lichtquellen über den Inspektionsbereich so verteilt angeordnet sind, dass jede Hohlkörper-Position auf dem Bahnabschnitt innerhalb des Inspektionsbereiches Licht aus einem weiten Winkelbereich empfängt, und dass eine den ganzen Inspektionsbereich erfassende Abbildungseinrichtung im Bild der durchlaufenden Hohlkörper ausser Durchlicht von den Lichtquellen auch aus den Hohlkörpen austretendes Sekundärlicht abbildet, das aus solchen Winkelbereichen in die durchsichtigen Hohlkörper eingetreten ist, bei denen es von den Hohlkörpern, in ihrer Eigenschaft als Lichtleiter, im Innern totalreflektiert wird.

Bei der Vorrichtung nach der Erfindung wird von dem Lichtleitereffekt Gebrauch gemacht, also von einem Effekt, der bisher hauptsächlich auf dem Gebiet der Glasfaseroptik angewendet wurde. Dazu wird der Teil des von den Lichtquellen ausgehenden Lichtes benutzt, der in einem solchen Winkel auf die Wand des hohlzylindrischen Bereichs des Hohlkörpers auftrifft und in diesen eintritt, dass er beim nächsten Auftreffen auf eine Grenzfläche zwischen dem Material des Hohlkörpers und der Luft total reflektiert wird. Er wird dann mit hoher Wahrscheinlichkeit erneut total reflektiert, so dass der Hohlkörper wie ein Lichtleiter wirkt. Andererseits tritt ein Teil dieses im Inneren des Hohlkörpers hin und her reflektierten Lichtes wieder aus diesem aus, wenn es auf eine Grenzfläche unter einem Winkel kleiner als der Grenzwinkel der Totalreflexion auftrifft. Dadurch wird der zu inspizierde Bereich des Hohlkörpers zu einer Sekundärlichtquelle. Je nach dem Winkel, den Flächen am Hohlkörper gegen die Zylinderachse des hohlzylindrischen Bereiches einnehmen, strahlen die Flächen des zur Sekundär-Lichtquelle gewordenen Hohlkörpers Licht in verschiedenen Intensitäten aus, so dass ein kontrastreiches Bild der Oberfläche des zylindrischen Bereiches erhältlich ist.

Dies ist ein ganz wesentlicher Vorteil gegenüber dem Stand der Technik, denn ein besonderer Zweck der Vorrichtung nach der Erfindung ist nicht das Beleuchten eines Bereiches des Hohlkörpers, in welchem dieser in jeder Beziehung vollkommen zylindrisch ist, sondern eines Bereiches wie der der Mündung, wo die Oberfläche des Hohlkörpers aufgrund beispielsweise von Gewindegängen nach aussen vorspringende Strukturen aufweist, die entsprechend der Erfindung durch den als Sekundär-Lichtquelle erscheinenden Hohlkörper mit hohem Kontrast beleuchtet, dargestellt und von der Abbildungseinrichtung abgebildet werden. Ebenso wirken Risse, Blasen u.s.w. im Inneren des Materials als Grenzflächen, an denen Licht austreten kann, wodurch auch innere Strukturfehler dieser Art erkennbar werden.

Vorteilhafte Ausgestaltungen der Erfindung bilden den Gegenstand von abhängigen Ansprüchen.

Zum Bewegen der Hohlkörper durch den Inspektionsbereich sind nach Anspruch 2 oder 3 Förderer, insbesondere Kreisförderer vorgesehen. An den Förderern kann nach Anspruch 4 eine Einrichtung vorhanden sein, welche die Hohlkörper auf ihrer Bahn durch den Inspektionsbereich rotieren lässt.

Nach Anspruch 5 sind die Lichtquellen vorzugsweise bezüglich ihrer Anordnung und/oder ihrer Lichtabgabe untereinander so abgestimmt, dass sie den Inspektionsbereich homogen beleuchten. Die Ansprüche 6 und 7 sagen aus, dass die Lichtquellen bezüglich ihres Abstands untereinander oder bezüglich der Lichtabgabe einstellbar sind. Mit diesen Einstellmöglichkeiten lässt sich beispielsweise bei zeitweisem Ausfall einer Lichtquelle die homogene Beleuchtung des Inspektionsbereiches aufrechterhalten.

Die Lichtquellen können nach Anspruch 8 in einem Bogen um den Inspektionbereich angeordnet werden. Besonders vorteilhaft sind nach Anspruch 9 Vorrichtungen, bei denen Lichtquellen mit gleicher Lichtabgabe gleichabständig auf einem Bogen in der Form eines Kreises um die Mitte des Inspektionsbereiches mit einem Radius merklich grösser als der halbe Abstand zwischen den äussersten Punkten des Inspektionsbereiches angeordnet oder nach Anspruch 10 Vorrichtungen, bei denen Lichtquellen mit gleicher Lichtabgabe auf einem Bogen in Form eines Ellipsenteils angeordnet sind, deren Brennpunkte die äusseren Punkte des Inspektionsbereiches, und ihre Abstände auf dem Ellipsenbogen umgekehrt proportional zu der Entfernung zum nächsten Brennpunkt sind. Durch die Verwendung von Lichtquellen mit gleicher Lichtabgabe in diesen Anordnungen wird erreicht, dass der Inspektionsbereich in seiner ganzen Ausdehnung mit Licht von annähernd homogener Intensität bestrahlt wird. Es sind jedoch auch andere Anordnungen der Lichtquellen, mit denen der Inspektionsbereich mit Licht von annähernd homogener Lichtintensität bestrahlt wird, anwendbar.

Weiter ist es nach Anspruch 11 vorteilhaft, zwischen den Lichtquellen und dem Inspektionsbereich eine Wand aus lichtundurchlässigem Material anzuordnen, die eine Lichtdurchtrittsöffnung in Form eines langgestreckten, quer zu den Längsachsen der Hohlkörper liegenden Schlitzes besitzen, dessen Höhe in der Höhe der zu inspizierenden Bereiche der Hohlkörper liegt und grösser ist als die Höhe dieser Bereiche. Nach Anspruch 12 bzw. 13 können diese Wände plan oder gekrümmt sein.

Nach Anspruch 14 lassen sich Lichtquellen verwenden, die stabförmige, zu der Längsachse des Hohlkörpers parallele Hochleistungslampen sind, bei denen nach Anspruch 15 die Mitte der Mittellinie der Länge des Schlitzes gegenüberliegt und deren Länge wenigstens gleich der zweifachen Schlitzhöhe ist. Nach Anspruch 16 können aber auch Lichtquellen verwendet werden, die langgestreckte, jeweils die Form eines Kreis- oder Ellipsenbogens aufweisende und quer zu der Flaschenlängsachse angeordnete Röhrenlampen sind.

In den Ansprüchen 17 bis 19 werden eine Reihe von Hochleistungslampen für die Verwendung in der Vorrichtung aufgezählt.

Durch das Vorsehen eines bogenförmigen Reflektors parallel zu einer bogenförmigen Lichtquellenanordnung lässt sich in der Ausgestaltung der Erfindung nach Anspruch 20 weitere Lichtenergie im Inspektionsbereich nutzbar machen. Diese Massnahme kann durch einen reflektierenden Überzug auf der den Lichtquellen zugewandten Innenseite der den Schlitz aufweisenden Wand noch unterstützt werden.

Weitere bogenförmige Reflektoren in der Ausgestaltung der Erfindung nach Anspruch 21 sorgen dafür, dass Licht, welches an den Enden des Schlitzes nach aussen entweichen würde, zurück in den Schlitz und damit zum Inspektionsbereich geworfen wird.

Der hohlzylindrische Bereich des Hohlkörpers gibt als Sekundärlichtquelle kontrastreich Oberflächenstrukturen darstellendes Licht ab. Das von den Lichtquellen ausgehende, den hohlzylindrischen Bereich des Hohlkörpers ohne Reflexionen durchstahlende Licht wirkt aber, falls es die Abbildungseinrichtung trifft, als störender Untergrund für die kontrastreiche Darstellung der Oberflächenstruktur des hohlzylindrischen Teils durch die Sekundärlichtquelle. Diese Störung wird verringert, wenn man nach Anspruch 22 und 23 die Abbildungseinrichtung so auf die Mitte des Inspektionsbereichs oder schräg gegen den Inspektionsbereich ausrichtet, dass sie zwar den zu inspizierenden Bereich der Hohlkörper im ganzen Inspektionsbereich, aber möglichst wenige der Lichtquellen direkt sieht.

Die Erfindung wird an Hand der Zeichnung näher erklärt, dabei zeigen:
- Fig. 1: eine Gesamtansicht einer Flaschenprüfmaschine, welche mit der Beleuchtungseinrichtung nach der Erfindung versehen ist,
- Fig. 2: eine perspektivische Darstellung der Vorrichtung mit Förderer und Abbildungseinrichtung zur Inspektion des Gewindebereichs von Flaschen,
- Fig. 3: eine Darstellung der Einfallswinkel-Bereiche, zu denen in einem Punkt der Oberfläche des Hohlkörpers einfallendes Licht zur Bildung einer Sekundärstrahlenquelll im Hohlkörper beiträgt,
- Fig. 4: eine schematische Darstellung einer bevorzugten Ausführung in einer Ansicht von oben
- Fig. 5: in einer schematischen Teilvorderansicht eine weitere Ausführungsform der Vorrichtung.
- Fig. 6: die Ausführungsform nach Fig. 5 in Draufsicht.

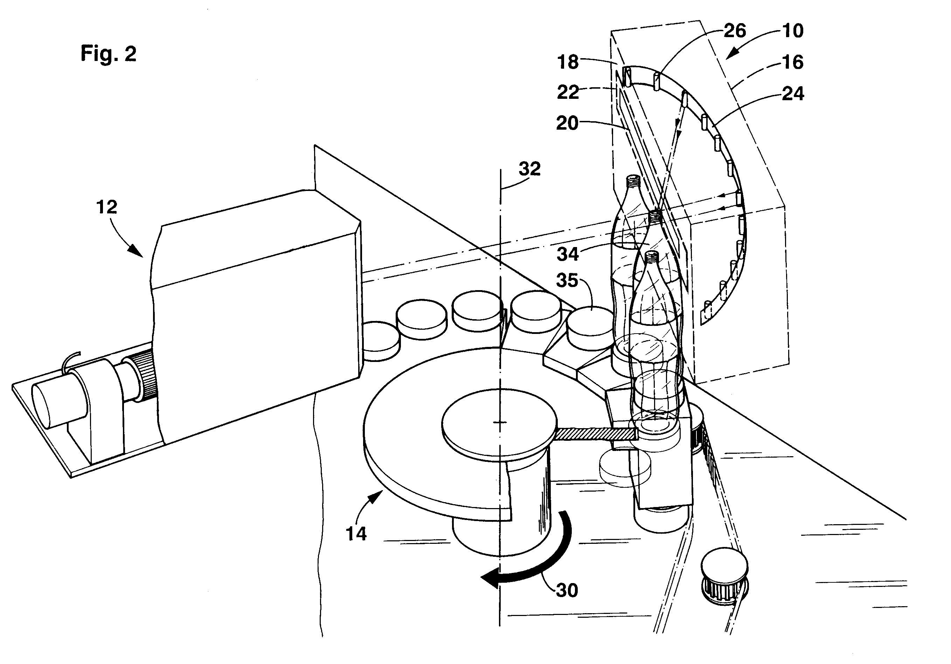

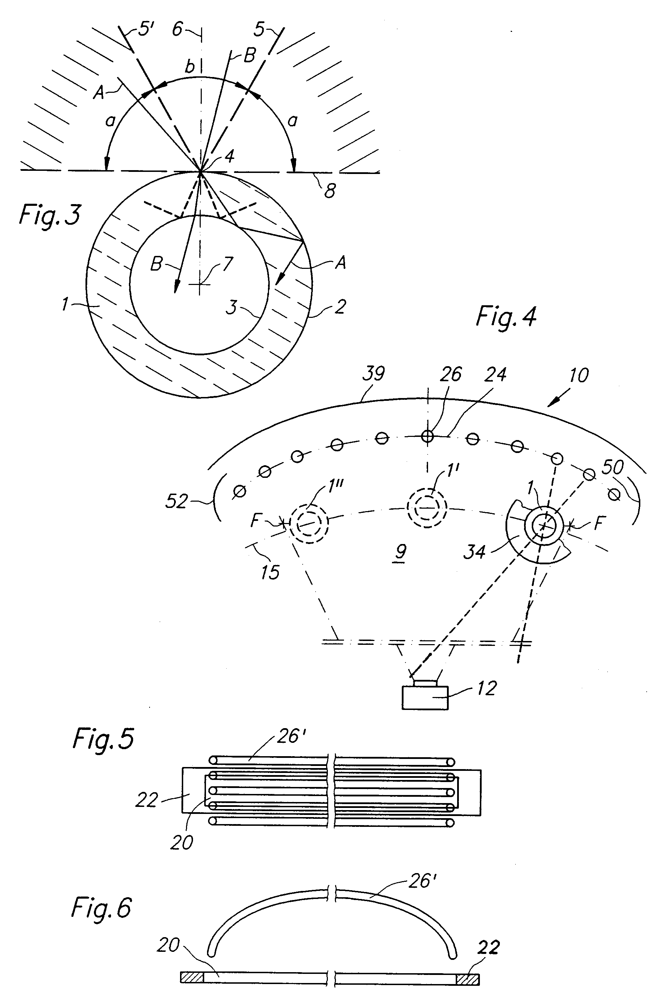

In der Zeichnung sind als Hohlkörper Flaschen 34 (Fig. 2) dargestellt, bei denen die Gewinde im Mündungsbereich, somit die Flaschenhälse 1 (Fig. 3) zu prüfen sind. Es wird daher im Folgenden teilweise der Ausdruck "Flaschen" statt Hohlkörper verwendet, wobei für beide dasselbe Beizeichen 34 angewendet wird. Diese Flaschen 34 werden innerhalb eines Inspektionsbereichs 9, der in der Fig. 4 dargestellt ist, geprüft.

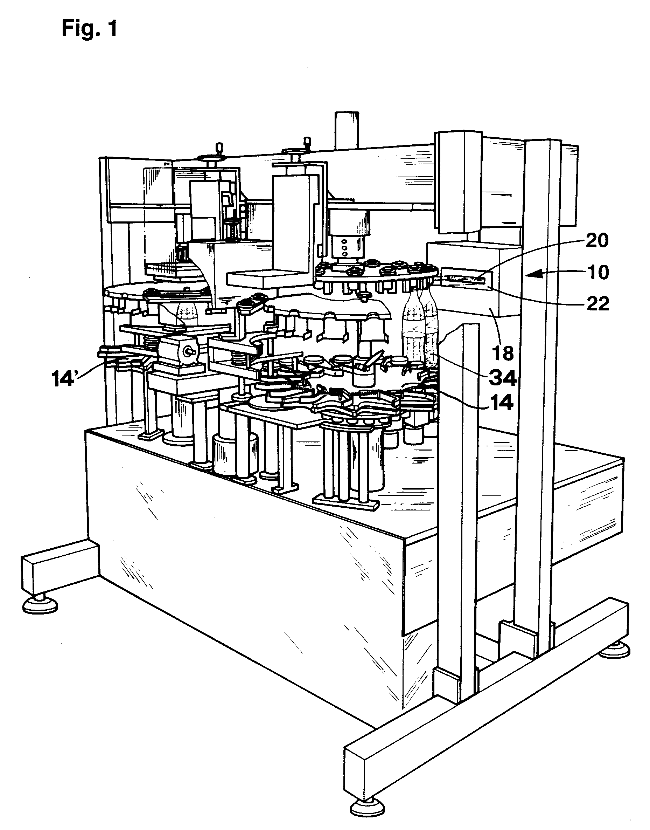

Die Fig. 1 zeigt eine Gesamtansicht einer Flaschenprüfmaschine, die eine mit 10 bezeichnete Beleuchtungseinrichtung für einen zu inspizierenden Mündungsbereich der Flaschen (34) aufweist.

Die Einrichtung 10 könnte statt zum Beleuchten des mit Gewinde versehenen Mündungsbereiches auch zum Beleuchten von anderen Bereichen von Flaschen 34 oder anderen mehr oder weniger durchsichtigen Körpern aller Art wie Hohlgläsern, Hohlkörpern aus PET, usw. benutzt werden. Die Maschine hat zwei Kreisförderer 14, welche die Flaschen (34) einander übergeben und sie auf zwei Kreisbahnen bewegen, damit verschiedene Prüfungen an den Flaschen 34 vorgenommen werden können. Statt der Kreisförderer 14 können auch Längsförderer in solchen Flaschenprüfmaschinen verwendet werden. Dem in Fig. 1 rechts dargestellten Kreisförderer 14 ist die Beleuchtungseinrichtung 10 zugeordnet, die an ihrer vorderen Wand 18 einen Schlitz 20 aufweist, der mit einer Glasplatte 22 abgedeckt ist. Eine eingehendere Darstellung einer erfindungsgemässen Anordnung findet sich in den Fig. 2 und 4.

Eine der Beleuchtungsvorrichtung 10 zugeordnete Abbildungseinrichtung 12, die in Fig. 1 nicht sichtbar ist, ist in Fig. 2 dargestellt. Fig. 2 zeigt ausserdem in vereinfachter Darstellung einen Kreisförderer 14, der Flaschen 34 fördert.

Die Beleuchtungseinrichtung 10 weist gemäss der Darstellung in Fig. 2 ein Gehäuse 16 auf. In ihm liegt zwischen in der Beleuchtungseinrichtung 10 vorhandenen Lichtquellen 26 und Flaschen 34 eine vordere Wand 18 aus lichtundurchlässigem Material. In der Wand 18 liegt ein langgestreckter, quer zu den Längsachsen der Flaschen 34 liegender Schlitz 20,
dessen Höhe in der Höhe des zu inspizierenden Bereichs der Flaschen 34 liegt und der grösser ist als die Höhe dieses Bereiches. Die Wand 18 kann plan oder gekrümmt sein. Üblicherweise ist der Schlitz 20 offen. Unter bestimmten Bedingungen kann er aber auch mit einer Platte 22 aus hitzebeständigem Glas bedeckt sein. In dem Gehäuse 16 ist ein Halter 24 für Lichtquellen 26 angebracht, bei denen es sich in der Ausführungsform nach Fig. 2 um stabförmige, zu der Flaschenlängsachse parallele Hochleistungslampen in Form von beispielsweise Xenon-Lampen handelt. Die Mitte dieser Lichtquellen 26 liegt der Längsmittellinie des Schlitzes 20 gegenüber und ihre Länge ist vorzugsweise etwa gleich der zweifachen Schlitzhöhe. Auf dem Halter 24 sind die Lichtquellen 26 im Bogen angeordnet, der hier ein Ellipsenbogen ist. Es könnte aber auch ein Kreisbogen sein. Die unteren bzw. oberen Enden der Lichtquellen 26 sind jeweils in gleicher Höhe angeordnet.

Die Lichtquellen 26 sind mit einer nicht dargestellten Stromquelle verbunden, an die sie in Parallelschaltung angeschlossen sind. Die Schaltungsanordnung oder die Stromquelle ist dabei so ausgebildet, dass die Lichtabgabe jeder Lichtquelle 26 einzeln oder aller Lichtquellen 26 gemeinsam einstellbar ist.

Der Kreisförderer 14 dreht sich in Richtung eines Pfeils 30 um eine Mittelachse 32 und bewegt dabei die Flaschen 34, die einzeln auf Drehtellern 35 vertikal abgestellt sind und um ihre Längsachse im Uhrzeigersinn an der Beleuchtungsvorrichtung 10 vorbei gedreht werden.

Grundsätzlich sind auch Vorrichtungen möglich, in der die Lichtquellen 26 auf der einen Seite des Bahnabschnittes 15 und der Achse der Hohlkörper 34 in wilkürlicher Weise angeordnet sind. Vorteilhaft ist es aber bei jeder Anordnung der Lichtquellen 26, diese so auszulegen, dass die Lichtintensität im Inspektionsbereich homogen ist.

In der Fig. 3 ist ein Flaschenhals 1 im Querschnitt dargestellt, der eine Aussenfläche 2 und eine Innenfläche 3 aufweist. Auf den Flaschenhals 1 trifft Licht aus allen Winkeln aus einem Halbraum heraus, dessen ebene Grenze durch die Linie 8 dargestellt wird. Es wird dasjenige Licht betrachtet, das in einem Punkt 4 auf der Aussenfläche 2 in den Flaschenhals eindringt. Es entstehen zwei räumliche, in der Fig. 3 eben dargestellte Winkelbereiche a, b. Das aus dem Winkelbereich a einfallende Licht trägt zur Bildung der Sekundärlichtquelle im Flaschenhals bei, das aus dem Winkelbereich b einfallende Licht trägt dagegen hauptsächlich zu dem vom Flaschenhals 1 direkt durchgelassenen Licht bei. Die Grenzstrahlen 5,5' trennen diese beiden Winkelbereiche voneinander. Die Grenzstrahlen 5,5' sind diejenigen Lichtstrahlen, die an der ersten Grenzfläche zwischen dem Flaschenmaterial und der Luft gerade den Grenzwinkel der totalen Reflexion erreichen. Dieser liegt bei Flaschenglas mit einem Brechungsindex von etwa n = 1,5 bei ungefähr 41^{o}.

Mit A ist ein Lichtstrahl gezeichnet, der im Punkt 4 aus dem Winkelbereich a kommend in den Flaschenhals 1 eintritt und an der nächsten Grenzfläche zwischen dem Flaschenmaterial und der Luft unter einem grösseren Winkel als dem Grenzwinkel der Totalreflexion auftrifft. Er erleidet mit grosser Wahrscheinlichkeit noch weitere Totalreflexionen im Flaschenhals 1, bis er aus diesem irgendwo wieder austritt, und trägt so zur Sekundärlichtquelle im Flaschenhals bei.

Mit B ist ein Lichtstrahl gezeichnet, der aus dem Winkelbereich b kommend am Punkt 4 in den Flaschenhals 1 eintritt. Er trifft unter einem kleineren Winkel als dem Grenzwinkel der Totalreflexion auf die Grenzfläche zwischen dem Flaschenmaterial und der Luft auf und wird mit nur geringem Reflexionsverlust durchgelassen. Somit liefert er einen Beitrag zu dem Durchlicht durch den Flaschenhals 1.

In der hier nicht gezeichneten räumlichen Darstellung entsteht ein zylindrischer Grenzstrahlkegel mit einer durch den Punkt 4 und die Flaschenachse 7 gehenden Achse 6. Die nicht gezeichneten Lichtquellen 26 beleuchten den Halbraum oberhalb des Flaschenhalses 1 (in Fig. 3 gesehen), die Linie 8 bildet dabei die Grenze des beleuchteten Bereiches. Wird beachtet, dass die vorzugsweise in einem Bogen um den Inspektionsbereich 9 (Fig.4) angeordneten Lichtquellen 26 den betrachteten Flaschenhals 1 vorzugsweise homogen beleuchten, so lässt sich erkennen, dass wesentlich mehr Licht in die Sekundärstrahlenquelle "Flaschenhals" 1 eintritt und von dieser in kontrastreicher Weise auch abgegeben wird als in die Durchstrahlung, welche die Kontrastbildung stört.

Um die über alle Punkte 4 der äusseren Oberfläche 2 in die Sekundarlichtquelle eingespeiste Lichtintensität und die störende Durchlassintensität abzuschätzen, müssten die Reflexionsverluste berücksichtigt und danach zur Gewinnung beider Intensitäten über alle Punkte 4 integriert werden.

In der Figur 4 wird das Schema einer Vorrichtung als Beispiel gezeigt. Auf einem Bahnabschnitt 15 befindet sich ein Inspektionsbereich 9, der von den Flaschen 34 (Fig.2) durchlaufen wird, von denen nur der Querschnitt des Flaschenhalses 1 gezeichnet ist. Der Flaschenhals 1 steht am rechten Ende des Inspektionsbereichs 9, nachdem er zuvor die Stellungen der gestrichelt gezeichneten Flaschenhälse 1' und 1'' durchlaufen hat. Diese Stellungen sind nun leer. Die Beleuchtungseinrichtung 10 besteht aus einem Halter 24, aus in einem elliptischen Bogen angeordneten Lichtquellen 26, aus einem elliptischen Reflektor 39, der in einer vom Inspektionsbereich 9 abgewandten Stellung in der Beleuchtungseinrichtung 10 angebracht ist und zwei an beiden Enden des Ellipsenbogens angebrachten bogenförmigen weiteren Reflektoren 50 und 52. Diese Reflektoren erhöhen die Lichtausbeute der Beleuchtungseinrichtung 10. Die Brennpunkte F der Ellipse der Lichtquellen 26 sind die Grenzen des Inspektionsbereiches 9. Die Reflektoren können auch auf die Aussen- oder Innenwände der Lichtquellen 26 aufgebracht werden.

Die Abbildungseinrichtung 12 ist auf der bezüglich des Inspektionsbereichs 9 gegenüberliegenden Seite der Beleuchtungseinrichtung 10 angebracht. Sie ist in diesem Beispiel auf die Mitte des Inspektionsbereichs 9 ausgerichtet und so eingestellt, dass sie gerade den ganzen Inspektionsbereich 9 erfasst. Die Abbildungseinrichtung 12 sieht zu dem dargestellten Zeitpunkt nur den in der rechten Stellung befindlichen Flaschenhals 1, da die Stellungen der Flaschenhälse 1' und 1'' ja bereits geräumt sind. Infolge ihrer Ausrichtung erfasst die Abbildungseinrichtung 12 nur sehr wenig Durchstrahllicht, sondern sieht den Flaschenhals 1 im wesentlichen nur als eine seine Oberflächenstukturen kontrastreich beleuchtende Sekundärlichtquelle. Dieselben Ueberlegungen lassen sich auch für die Stellungen 1' und 1'' durchführen und für sämtliche Zwischenstellungen im Inspektionbereich 9. Damit ist gezeigt, dass in jeder Position im Inspektionbereich 9 Licht unter solchen Einfallswinkeln empfangen wird, bei dem der Hohlkörper 34 sich in Bezug auf die Abbildungsvorrichtung 12 als Sekundärlichtquelle verhält.

Als Lichtquellen 26, 26' können Hochleistungslampen, wie Xenon-Lampen, Halogen- oder Natriumdampflampen benutzt werden. Ausserdem kann es sich um Hochdruck- oder um Niederdrucklampen handeln. Wesentlich ist, dass die Lampen eine hohe Lichtintensität liefern.

Weiter kann der Bogen, auf dem die Lichtquellen 26 angeordnet sind, auch ein Halbkreis um den Mittelpunkt des Inspektionsbereichs 9 sein, mit einem Radius merklich grösser als der halbe Abstand zwischen den äussersten Punkten des Inspektionsbereichs.

Die Fig. 5a und 5b zeigen in schematischer Darstellung in einer Vorderansicht bzw. in Draufsicht eine vorteilhafte Ausführungsform langgestreckter Lichtquellen 26', die jeweils die Form einer Halbellipse aufweisen und quer zu der Flaschenlängsachse angeordnete Röhrenlampen sind. Auch die Lichtquellen 26' sind in derartigen gegenseitigen Abständen angeordnet, dass sie im Inspektionsbereich 9 (Fig.4) eine homogene Lichtintensität liefern.

Damit eine einzige Abbildungseinrichtung 12 den gesamten Umfang des Flaschenhalses 1 erfassen kann, wird vorteilhafterweise die Flasche 34 während ihres Durchlaufs durch den Inspektionsbereich 9 gedreht.

Die erfindungsgemässe Vorrichtung erzeugt eine Sekundärlichtquelle in den zu inspizierenden, im wesentlichen hohlzylindrischen Bereichen von Hohlkörpern 34, beispielsweise in Flaschenhälsen 1, und unterdrückt die störende Durchlassstrahlung. Dies ermöglicht eine die Oberflächenstrukturen und dergleichen kontrastreich wiedergebende Darstellung der zu inspizierenden Bereiche mit Hilfe der Abbildungseinrichtung 12.

## Patentansprüche

1. Vorrichtung zum Inspizieren eines im wesentlichen hohlzylindrischen Bereiches von entlang einer Bahn bewegten, durchsichtigen Hohlkörpern (34), wobei die Hohlkörper auf einem Bahnabschnitt (15) einen Inspektionsbereich (9) durchlaufen, mit einem auf der einen Seite des Bahnabschnittes (15) und der Achse (7) der Hohlkörper befindlichen, auf den Inspektionsbereich (9) gerichteten Lichtempfänger (12) und einer auf der gegenüberliegenden Seite des Bahnabschnittes angeordneten, den Inspektionsbereich bestrahlende Lichquellen (26, 26') enthaltenden Beleuchtungseinrichtung (10),
dadurch **gekennzeichnet,** dass die Lichtquellen (26, 26') über den Inspektionsbereich (9) so verteilt angeordnet sind, dass jede Hohlkörper-Position (1, 1', 1'') auf dem Bahnabschnitt (15) innerhalb des Inspektionsbereiches (9) Licht aus einem weiten Winkelbereich (a + b) empfängt, und dass eine den ganzen Inspektionsbereich (9) erfassende Abbildungseinrichtung (12) im Bild der durchlaufenden Hohlkörper (34) ausser Durchlicht (B) von den Lichtquellen (26, 26) auch aus den Hohlkörpern austretendes Sekundärlicht (A) abbildet, das aus solchen Winkelbereichen (a) in die durchsichtigen Hohlkörper eingetreten ist, bei denen es von den Hohlkörpern, in ihrer Eigenschaft als Lichtleiter, im Innern totalreflektiert wird.

2. Vorrichtung nach Anspruch 1, dadurch gekennzeichnet, dass zum Bewegen der Hohlköper (34) ein Förderer (14) vorgesehen ist.

3. Vorrichtung nach Anspruch 2, dadurch gekennzeichnet, dass der Förderer ein Kreisförderer (14) ist.

4. Vorrichtung nach einem der Ansprüche 2 bis 3, dadurch gekennzeichnet, dass an dem Förderer (14) eine Einrichtung vorhanden ist, welche die Hohlkörper (34) auf ihrer Bahn durch den Inspektionsbereich (9) rotieren lässt.

5. Vorrichtung nach Anspruch 1, dadurch gekennzeichnet, dass die Lichtquellen (26, 26') bezüglich ihrer Anordnung und/oder ihrer Lichtabgabe so abgestimmt sind, dass sie den Inspektionsbereich (9) homogen beleuchten.

6. Vorrichtung nach Anspruch 5, dadurch gekennzeichnet, dass der Abstand zwischen den Lichtquellen (26, 26') einstellbar ist.

7. Vorrichtung nach Anspruch 5, dadurch gekennzeichnet, dass die Lichtabgabe jeder Lichtquelle (26, 26') einstellbar ist.

8. Vorrichtung nach Anspruch 1 oder 5, dadurch gekennzeichnet, dass die Lichtquellen (26,26') auf einem Bogen um den Inspektionsbereich (9) angeordnet sind.

9. Vorrichtung nach Anspruch 8, dadurch gekennzeichnet, dass die Lichtquellen (26,26') mit gleicher Lichtabgabe gleichabständig auf einem Bogen in Form eines Kreises um die Mitte des Inspektionsbereichs (9) angeordnet sind, wobei der Radius des Kreises merklich grösser ist als der halbe Abstand zwischen den äussersten Punkten des Inspektionsbereichs (9).

10. Vorrichtung nach Anspruch 8, dadurch gekennzeichnet, dass Lichtquellen (26, 26') mit gleicher Lichtabgabe auf einem Bogen in Form eines Ellipsenteils angeordnet sind, wobei die Brennpunkte (F) der Ellipse die äusseren Punkte des Inspektionsbereiches (9) und die Abstände der Lichtquellen auf dem Ellipsenbogen umgekehrt proportional zu der Entfernung zum jeweils näheren Brennpunkt (F) der Ellipse sind.

11. Vorrichtung nach Anspruch 1, dadurch gekennzeichnet, dass zwischen den Lichtquellen (26, 26') und dem Inspektionsbereich (9) eine Wand (18) aus lichtundurchlässigem Material angeordnet ist, die eine Lichtdurchtrittsöffnung in Form eines langgestreckten, quer zu den Längsachsen der Hohlkörper (34) liegenden Schlitzes (20) besitzt, dessen Höhe in der Höhe der zu inspizierenden Bereiche der Hohlkörper (34) liegt und grösser als die Höhe dieser Bereiche ist.

12. Vorrichtung nach Anspruch 11, dadurch gekennzeichnet, dass die Wand (18) plan ist.

13. Vorrichtung nach Anspruch 11, dadurch gekennzeichnet, dass die Wand (18) gekrümmt ist.

14. Vorrichtung nach Ansprch 1, dadurch gekennzeichnet, dass die Lichtquellen (26) stabförmige, zur Achse der hohlzylindrischen Bereiche der Hohlkörper (34) parallel angeordnete Lampen sind.

15. Vorrichtung nach Anspruch 11 und 14, dadurch gekennzeichnet, dass die Lichtquellen (26) parallele Hochleistungslampen sind, deren Mitte der Längsmittellinie des Schlitzes (20) gegenüberliegt und deren Länge wenigstens gleich der zweifachen Schlitzhöhe ist.

16. Vorrichtung nach den Ansprüche 9 oder 10, dadurch gekennzeichnet, dass die Lichtquellen (26') langgestreckte, jeweils die Form des Kreis- oder Ellipsenbogens aufweisende und quer zu den Achsen der Hohlkörper (34) angeordnete Röhrenlampen sind.

17. Vorrichtung nach Anspruch 1, dadurch gekennzeichnet, dass die Lichtquellen (26, 26') Xenon-Lampen sind.

18. Vorrichtung nach Anspruch 1, dadurch gekennzeichnet, dass die Lichtquellen (26, 26') Halogen-Lampen sind.

19. Vorrichtung nach Anspruch 1, dadurch gekennzeichnet, dass die Lichtquellen (26, 26') Leuchtstofflampen sind.

20. Vorrichtung nach Anspruch 8, dadurch gekennzeichnet, dass auf der von den Hohlkörpern (34) abgewandten Seite des Bogens der Lichtquellen (26, 26') ein bogenförmiger Reflektor (39) vorgesehen ist.

21. Vorrichtung nach Anspruch 20, dadurch gekennzeichnet, dass an den beiden Enden des Bogens der Lichtquellen (26, 26') je ein bogenförmiger weiterer Reflektor (50, 52) angeordnet ist.

22. Vorrichtung nach Anspruch 1, dadurch gekennzeichnet, dass die Abbildungseinrichtung (12) senkrecht auf die Mitte des Inspektionsbereichs (9) gerichtet ist.

23. Vorrichtung nach Anspruch 1, dadurch gekennzeichnet, dass die Abbildungseinrichtung (12) schräg auf den Inspektionsbereich (9) gerichtet ist.

## Claims

1. A device for inspecting a substantially hollow, cylindrical region of transparent hollow bodies (34) moved along a track, wherein the hollow bodies (34) pass through an inspection region (9) on a track section (15), with a light receiver (12) oriented towards the inspection region (9) and located on one side of the track section (15) and on the axis of the hollow bodies (34), and with an illuminating device (10) disposed on the opposite side of the track section, containing light sources (26, 26') and irradiating the inspection region (9), characterised in that the light sources (26, 26') are arranged distributed over the inspection region (9) so that each hollow body position (1, 1', 1'') on the track section (15) within the inspection region (9) receives light from a broad angular range (a + b), and that in addition to transmitted light (B) from the light sources (26, 26') an imaging device (12) which detects the entire inspection region (9) also maps the secondary light (A) emerging from the hollow bodies which has entered the transparent hollow bodies from angular ranges (a) such that it is totally internally reflected by the hollow bodies in their capacity as wave-guides.

2. A device according to claim 1, characterised in that a conveyor (14) is provided for moving the hollow bodies (34).

3. A device according to claim 2, characterised in that the conveyor is a circular conveyor (14).

4. A device according to one of claims 2 to 3, characterised in that a device is present on the conveyor (14) which allows the hollow bodies (34) to rotate on their path through the inspection region (9).

5. A device according to claim 1, characterised in that the light sources (26, 26') are balanced with respect to their arrangement and/or light output so that they illuminate the inspection region (9) homogeneously.

6. A device according to claim 5, characterised in that the distance between the light sources (26, 26') is adjustable.

7. A device according to claim 5, characterised in that the light output of each light source (26, 26') is adjustable.

8. A device according to claim 1 or 5, characterised in that the light sources (26, 26') are arranged in an arc around the inspection region (9).

9. A device according to claim 8, characterised in that the light sources (26, 26') are arranged equidistantly with the same light output in an arc in the form of a circle around the centre of the inspection region (9), wherein the radius of the circle is appreciably larger than half the distance between the outermost points of the inspection region (9).

10. A device according to claim 8, characterised in that light sources (26, 26') are arranged with the same light output in an arc in the form of a partial ellipse, wherein the focal points (F) of the ellipse are the outermost points of the inspection region (9) and the distances between the light sources on the elliptical arc are inversely proportional to the distance to the respective nearest focal point (F) of the ellipse.

11. A device according to claim 1, characterised in that a wall (18) of material opaque to light is arranged between the light sources (26, 26') and the inspection region (9), which wall has an aperture for light to pass through in the form of an elongated slit (20) situated transversely to the longitudinal axes of the hollow bodies (34), the vertical dimension of which aperture is at the same height as the height of the regions of the hollow bodies (34) to be inspected and is larger than the vertical dimension of these regions.

12. A device according to claim 11, characterised in that the wall (18) is plane.

13. A device according to claim 11, characterised in that the wall (18) is curved.

14. A device according to claim 1, characterised in that the light sources (26, 26') are rod-shaped lamps arranged parallel to the axis of the hollow cylindrical regions of the hollow bodies (34).

15. A device according to claims 11 and 14, characterised in that the light sources (26) are parallel high-power lamps, the middle one of which is situated opposite to the line which longitudinally bisects the slit (20) and the length of which is at least equal to twice the height of the slit.

16. A device according to claims 9 or 10, characterised in that the light sources (26') are elongated tubular lamps, each having the form of a circular or elliptical arc, and disposed transversely to the axes of the hollow bodies (34).

17. A device according to claim 1, characterised in that the light sources (26, 26') are xenon lamps.

18. A device according to claim 1, characterised in that the light sources (26, 26') are halogen lamps.

19. A device according to claim 1, characterised in that the light sources (26, 26') are fluorescent tubes.

20. A device according to claim 8, characterised in that an arc-shaped reflector is provided on the side of the arc of the light sources (26, 26') remote from the hollow bodies (34).

21. A device according to claim 20, characterised in that an additional arc-shaped reflector (50, 52) is disposed at each of the two ends of the arc comprising the light sources (26, 26').

22. A device according to claim 1, characterised in that the imaging device (12) is aligned vertically at the centre of the inspection region (9).

23. A device according to claim 1, characterised in that the imaging device (12) is aligned obliquely at the centre of the inspection region (9).

## Revendications

1. Dispositif pour inspecter une partie essentiellement en forme de cylindre creux de corps creux transparents (34), qui se déplacent le long d'une trajectoire et qui, dans une section de trajectoire (15), traversent une zone d'inspection (9), comportant un récepteur de lumière (12) situé d'un côté de la section de trajectoire (15) et de l'axe (7) des corps creux et dirigé vers la zone d'inspection (9), et un dispositif d'éclairement (10) qui est disposé sur le côté opposé de la section de trajectoire et contient des sources de lumière (26, 26') éclairant la zone d'inspection,
caractérisé en ce que les sources de lumière (26, 26') sont disposées en étant réparties au-dessus de la zone d'inspection (9) de telle sorte que chaque position (1, 1', 1'') d'un corps creux sur la section de trajectoire (15) à l'intérieur de la zone d'inspection (9) reçoit de la lumière provenant d'une plage angulaire étendue (a+b), et qu'un dispositif de formation d'images (12), qui détecte l'ensemble de la zone d'inspection (9), forme, dans l'image des corps creux (34) qui circulent, non seulement l'image de la lumière transmise (B) délivrée par les sources de lumière (26, 26'), mais également une lumière secondaire (A), qui a pénétré dans les corps creux transparents et en sort, à partir de plages angulaires (a), dans lesquelles elle subit une réflexion totale à l'intérieur des corps creux, en raison de leur caractéristique de guides de lumière.

2. Dispositif selon la revendication 1, caractérisé en ce qu'un convoyeur (14) est prévu pour déplacer les corps creux (34).

3. Dispositif selon la revendication 2, caractérisé en ce que le convoyeur est un convoyeur circulaire (14).

4. Dispositif selon l'une des revendications 2 et 3, caractérisé en ce que pour le convoyeur (14) il est prévu un dispositif qui fait tourner les corps creux (34) sur leur trajectoire à travers la zone d'inspection (9).

5. Dispositif selon la revendication 1, caractérisé en ce que les sources de lumière (26, 26'), en ce qui concerne leur disposition et/ou leur émission de lumière sont réglées de telle sorte qu'elles éclairent d'une manière homogène la zone d'inspection (9).

6. Dispositif selon la revendication 5, caractérisé en ce que la distance entre les sources de lumière (26, 26') est réglable.

7. Dispositif selon la revendication 5, caractérisé en ce que l'émission de lumière de chaque source de lumière (26, 26') est réglable.

8. Dispositif selon la revendication 1 ou 5, caractérisé en ce que les sources de lumière (26, 26') sont disposées sur un arc autour de la zone d'inspection (9).

9. Dispositif selon la revendication 8, caractérisé en ce que les sources de lumière (26, 26'), qui délivrent une même émission de lumière, sont disposées de manière équidistante sur un arc possédant la forme d'un cercle autour du centre de la zone d'inspection (9), le rayon du cercle étant nettement supérieur à la moitié de la distance entre les points les plus extérieurs de la zone d'inspection (9).

10. Dispositif selon la revendication 8, caractérisé en ce que les sources de lumière (26, 26'), qui délivrent la même émission de lumière, sont disposées sur un arc ayant la forme d'une partie d'ellipse, dont les foyers (F) sont les points extérieurs de la zone d'inspection (9), tandis que les distances des sources de lumière sur l'arc d'ellipse sont inversement proportionnelles à la distance par rapport au foyer respectif (F), le plus proche, de l'ellipse.

11. Dispositif selon la revendication 1, caractérisé en ce qu'entre les sources de lumière (26, 26') et la zone d'inspection (9) est disposée une paroi (18) formée d'un matériau opaque, qui possède une ouverture de passage de la lumière se présentant sous la forme d'une fente allongée (20), qui s'étend transversalement par rapport aux axes longitudinaux des corps creux (34) et dont le niveau se situe au niveau des zones à inspecter des corps creux (34) et dont la hauteur est supérieure à la hauteur de ces zones.

12. Dispositif selon la revendication 11, caractérisé en ce que la paroi (18) est plane.

13. Dispositif selon la revendication 11, caractérisé en ce que la paroi (18) est cintrée.

14. Dispositif selon la revendication 1, caractérisé en ce que les sources de lumière (26) sont des lampes de forme tubulaire, qui sont parallèles à l'axe des zones en forme de cylindres creux des corps creux (24).

15. Dispositif selon les revendications 11 et 14, caractérisé en ce que les sources de lumière (26) sont des lampes de grande puissance parallèles, dont le centre est situé en vis-à-vis de l'axe médian longitudinal de la fente (20) et dont la longueur est au moins égale au double de la hauteur des fentes.

16. Dispositif selon la revendication 9 ou 10, caractérisé en ce que les sources de lumière (26') sont des lampes tubulaires allongées, qui possèdent respectivement la forme de l'arc de cercle ou d'ellipse et sont disposées transversalement par rapport aux axes des corps creux (34).

17. Dispositif selon la revendication 1, caractérisé en ce que les sources de lumière (26, 26') sont des lampes à xénon.

18. Dispositif selon la revendication 1, caractérisé en ce que les sources de lumière (26, 26') sont des lampes à halogène.

19. Dispositif selon la revendication 1, caractérisé en ce que les sources de lumière (26, 26' ) sont des lampes fluorescentes.

20. Dispositif selon la revendication 8, caractérisé en ce qu'un réflecteur de forme arquée (39) est prévu sur le côté, tourné à l'opposé des corps creux (34), de l'arc des sources de lumière (26, 26').

21. Dispositif selon la revendication 20, caractérisé en ce que respectivement un autre réflecteur de forme arquée (50, 51) est disposé aux deux extrémités de l'arc des sources de lumière (26, 26').

22. Dispositif selon la revendication 20, caractérisé en ce que le dispositif de formation d'images (12) est orienté perpendiculairement au centre de la zone d'inspection (9).

23. Dispositif selon la revendication 1, caractérisé en ce que le dispositif de formation d'images (12) est dirigé obliquement sur la zone d'inspection (9).
